(19) Europäisches Patentamt / European Patent Office / Office européen des brevets

(11) **EP 4 606 304 A1**

(12) **EUROPEAN PATENT APPLICATION**
published in accordance with Art. 153(4) EPC

(43) Date of publication:
**27.08.2025 Bulletin 2025/35**

(21) Application number: **23879684.1**

(22) Date of filing: **11.10.2023**

(51) International Patent Classification (IPC):
***A61B 5/1455*** (2006.01)

(52) Cooperative Patent Classification (CPC):
**A61B 5/1455**

(86) International application number:
**PCT/JP2023/036931**

(87) International publication number:
**WO 2024/085041 (25.04.2024 Gazette 2024/17)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **19.10.2022 JP 2022167840**

(71) Applicant: **OMRON Corporation
Kyoto 600-8530 (JP)**

(72) Inventors:
• **KUBO, Mitsuaki
Kyoto-shi, Kyoto 600-8530 (JP)**
• **KOIZUMI, Masayuki
Kyoto-shi, Kyoto 600-8530 (JP)**

• **KAWACHI, Masahiro
Kyoto-shi, Kyoto 600-8530 (JP)**
• **MATSUI, Yuki
Kyoto-shi, Kyoto 600-8530 (JP)**
• **YAMAMOTO, Kazuo
Kyoto-shi, Kyoto 600-8530 (JP)**
• **KIGUCHI, Tetsuya
Kyoto-shi, Kyoto 600-8530 (JP)**
• **ARAKAWA, Masayuki
Kyoto-shi, Kyoto 600-8530 (JP)**
• **OHASHI, Suguru
Kyoto-shi, Kyoto 600-8530 (JP)**

(74) Representative: **SONN Patentanwälte GmbH & Co
KG
Riemergasse 14
1010 Wien (AT)**

(54) **OXYGEN SATURATION MEASUREMENT DEVICE, OXYGEN SATURATION MEASUREMENT METHOD, AND OXYGEN SATURATION MEASUREMENT PROGRAM**

(57) An oxygen saturation measuring device determines a first light projection timing at which a first light emitting element projects red light and a second light projection timing at which a second light emitting element projects infrared light based on a peak timing and a bottom timing of a third pulse wave signal corresponding to a light intensity of received light of reference light having a wavelength at which an absorption coefficient of oxidized hemoglobin and an absorption coefficient of reduced hemoglobin are higher than those of the red light and the infrared light in one pulsation, causes the first light emitting element to project the red light at the first light projection timing, causes the second light emitting element to project the infrared light at the second light projection timing, and measures oxygen saturation of an artery based on a first pulse wave signal output from a light receiving element according to the red light projected at the first light projection timing and a second pulse wave signal output from the light receiving element according to the infrared light projected at the second light projection timing.

# FIG.5

**Description**

Technical Field

The present disclosure relates to an oxygen saturation measuring device, an oxygen saturation measuring method, and an oxygen saturation measuring program.

Background Art

Hitherto, as an example of a method of measuring oxygen saturation ($S_PO_2$) in arterial blood, a measuring method using an absorption spectroscopy as in Japanese Patent Application Laid-Open (JP-A) No. 2008-167868 is known. In JP-ANo. 2008-167868, red light and infrared light having wavelengths different from each other are projected to arterial blood, and transmitted light or reflected light corresponding to each light is received by a light receiving element. Hereinafter, for convenience of description, light received by the light receiving element, such as the transmitted light and the reflected light, is collectively referred to as "received light".

Specifically, amplitudes of a plurality of pulse wave signals are calculated for each of the red light and the infrared light from a maximum value and a minimum value acquired from the pulse wave signals of the received light. In order to increase the number of acquired pieces of data for amplitude calculation, usually, a light projection interval and a light reception interval of the red light and the infrared light for measurement are repeated a plurality of times at an interval shorter than one heartbeat interval of an artery to be measured.

A fluctuation component (AC) and a fixed component (DC) of the pulse wave signal are calculated from the plurality of calculated amplitudes, and a perfusion index (PI value) of each of the red light and the infrared light is calculated from the calculated fluctuation component and fixed component. Then, the oxygen saturation as a measured value can be calculated by introducing a ratio of the PI value of the red light to the PI value of the infrared light (that is, an absorbance ratio) into a preset calculation formula for oxygen saturation calculation.

Further, in JP-A No. 2008-167868, predetermined pulse wave information such as a sampling number and a pulse rate of the pulse wave signal obtained by one pulsation is obtained from a pulse wave signal output corresponding to a light intensity of the received light. In JP-A No. 2008-167868, as an interval of a light emission time of a light emitting element is set based on the obtained pulse wave information, the oxygen saturation can be measured with high accuracy and power consumption of an oxygen saturation measuring device can be reduced.

Citation Literature

Patent Literature

Patent Literature 1: JP-A No. 2008-167868

SUMMARY OF INVENTION

Technical Problem

Here, since a signal-to-noise (SN) ratio decreases as a blood flow is relatively low perfusion, that is, the light intensity of the pulse wave signal of the received light is low, accuracy in calculation of the amplitude of the pulse wave signal decreases. In this regard, in JP-A No. 2008-167868, only the pulse wave signals of the received light of the red light and the infrared light are used for measurement. Therefore, in a case in which the SN ratio of the pulse wave signal of the received light is low, accuracy of the pulse wave information obtained from the pulse wave signal also decreases, or necessary pulse wave information cannot be obtained. As a result, it is difficult to set the interval of the light emission time based on the pulse wave information.

The present disclosure has been made focusing on the above, and provides an oxygen saturation measuring device, an oxygen saturation measuring method, and an oxygen saturation measuring program capable of improving measurement accuracy.

Solution to Problem

An oxygen saturation measuring device according to a first aspect of the present disclosure includes: a sensor unit including a first light emitting element that projects red light to an artery, a second light emitting element that projects infrared light to the artery, a third light emitting element that projects reference light, which has a wavelength at which an absorption coefficient of oxidized hemoglobin and an absorption coefficient of reduced hemoglobin are higher than those of red light and infrared light to the artery, and a light receiving element that receives, as received light, transmitted light or reflected light corresponding to each of the projected red light, the projected infrared light, and the projected reference light, and outputs a first pulse wave signal corresponding to a light intensity of the received light of the red light, a second pulse wave signal corresponding to a light intensity of the received light of the infrared light, and a third pulse wave signal corresponding to a light intensity of the received light of the reference light; and a processor electrically connected to the first light emitting element, the second light emitting element, and the light receiving element and configured to determine, based on a peak timing and a bottom timing of the third pulse wave signal in one pulsation, a first light projection timing at which the first light emitting element projects the red light and a second light projection timing at which the second light emitting element projects the infrared light, cause the first light emitting element to project the red light at the determined first light projection timing, cause the second light emitting element to project the infrared light at the determined second light projection timing, and calculate oxygen saturation based on the first pulse wave signal acquired according to the light intensity of the received light of the red light projected at the first light projection timing and the second pulse wave signal acquired according to the light intensity of the received light of the infrared light projected at the second light projection timing to measure the oxygen saturation of the artery.

According to the first aspect, the wavelength of the reference light projected to the artery is a wavelength at which the absorption coefficient of the oxidized hemoglobin and the absorption coefficient of the reduced hemoglobin are higher than those of the red light and the infrared light. Therefore, a signal-to-noise (SN) ratio of the third pulse wave signal corresponding to the reference light is higher than those of the first pulse wave signal corresponding to the red light and the second pulse wave signal corresponding to the infrared light. That is, the SN ratio of the pulse wave signal of the received light depends on the wavelength of the projected light.

Therefore, by projecting the red light for measurement at the first light projection timing determined according to the peak timing and the bottom timing of the third pulse wave signal, calculation accuracy for an amplitude of the first pulse wave signal of the received light corresponding to the red light is improved. Similarly, since the infrared light for measurement is projected at the second light projection timing determined according to the peak timing and the bottom timing, the calculation accuracy of an amplitude of the second pulse wave signal of the received light corresponding to the infrared light is improved. As a result, the measurement accuracy for the oxygen saturation measured based on the first pulse wave signal and the second pulse wave signal is improved.

In a second aspect, according to the first aspect, the processor is configured to, based on the peak timing and the bottom timing of the acquired third pulse wave signal, calculate a heartbeat interval of a predicted pulsation of the third pulse wave signal expected to arrive and a predicted peak timing and a predicted bottom timing in the predicted pulsation, and determine the first light projection timing and the second light projection timing in the predicted pulsation based on the predicted peak timing and the predicted bottom timing that are calculated.

With the above configuration, the first light projection timing and the second light projection timing can be determined with high accuracy.

In a third aspect, according to the second aspect, the processor is configured to determine the first light projection timing and the second light projection timing in each of a first maximum side light projection period from a timing at which 2/3 of the heartbeat interval of the predicted pulsation elapses from the peak timing in a pulsation immediately before the predicted pulsation to the predicted peak timing, and a first minimum side light projection period from the predicted bottom timing of the predicted pulsation to a timing at which 1/3 of the heartbeat interval of the predicted pulsation elapses from the predicted bottom timing.

With the above configuration, the first light projection timing and the second light projection timing can be determined with higher accuracy.

In a fourth aspect, according to any one of the first to third aspects, the processor is configured to set a maximum side threshold value of an amplitude based on a maximum amplitude of the third pulse wave signal acquired at the peak timing, set a minimum side threshold value of the amplitude based on a minimum amplitude of the third pulse wave signal acquired at the bottom timing, and determine the first light projection timing and the second light projection timing in each of a second maximum side light projection period in which the amplitude is equal to or higher than the maximum side threshold value and a second minimum side light projection period in which the amplitude is equal to or lower than the minimum side threshold value in the pulsation of the third pulse wave signal.

With the above configuration, the first light projection timing and the second light projection timing can be determined with high accuracy.

In a fifth aspect, according to the fourth aspect, the maximum side threshold value is 2/3 or more of a difference between the maximum amplitude and the minimum amplitude, and the minimum side threshold value is 1/3 or less of the difference between the maximum amplitude and the minimum amplitude.

With the above configuration, the first light projection timing and the second light projection timing can be determined with higher accuracy.

In a sixth aspect, according to any one of the first to fifth aspects, the processor is configured to calculate a coefficient of variation representing a state of the pulsation based on a heartbeat interval of the acquired third pulse wave signal, execute processing 1 in a case in which the calculated coefficient of variation is equal to or less than a preset threshold value, and execute processing 2 in a case in which the coefficient of variation exceeds the threshold value.

(Processing 1)

Processing of based on the peak timing and the bottom timing of the acquired third pulse wave signal,000000 calculating a heartbeat interval of a predicted pulsation of the third pulse wave signal expected to arrive and a predicted peak timing and a predicted bottom timing in the predicted pulsation, and determining the first light projection timing and the second light projection timing in the predicted pulsation based on the predicted peak timing and the predicted bottom timing that are calculated.

(Processing 2)

Processing of setting a maximum side threshold value of an amplitude based on a maximum amplitude of the third pulse wave signal acquired at the peak timing, setting a minimum side threshold value of the amplitude based on a minimum amplitude of the third pulse wave signal acquired at the bottom timing, and determining the first light projection timing and the second light projection timing in each of a second maximum side light projection period in which the amplitude is equal to or higher than the maximum side threshold value and a second minimum side light projection period in which the amplitude is equal to or lower than the minimum side threshold value in the pulsation of the third pulse wave signal.

With the above configuration, preferable oxygen saturation measurement processing can be selected according to the state of the pulsation of a subject.

In a seventh aspect, according to the sixth aspect, the threshold value of the coefficient of variation is 0.1 or less.

With the above configuration, switching accuracy of the oxygen saturation measurement processing can be improved.

In an eighth aspect, the oxygen saturation measuring device according to the first to seventh aspects is a wearable device that is wearable by the subject.

Here, in the case of a measurement device such as a wearable device driven by a relatively compact primary battery or secondary battery, it is difficult to increase light projection power per one time because it is necessary to avoid shortening of a continuous use time. However, with the above configuration, a calculation load is reduced, and thus, the input power can be reduced. Therefore, it is possible to implement a wearable device capable of achieving a long life.

An oxygen saturation measuring method according to a ninth aspect of the disclosure includes: projecting, to an artery, reference light having a wavelength at which an absorption coefficient of oxidized hemoglobin and an absorption coefficient of reduced hemoglobin are higher than those of red light and infrared light; receiving, as received light, transmitted light or reflected light corresponding to the projected reference light; acquiring a third pulse wave signal corresponding to a light intensity of the received light of the reference light; based on a peak timing and a bottom timing of the acquired third pulse wave signal in one pulsation, determining a first light projection timing at which the red light is projected and a second light projection timing at which the infrared light is projected; projecting the red light at the determined first light projection timing; projecting the infrared light at the determined second light projection timing; and calculating oxygen saturation based on a first pulse wave signal acquired according to a light intensity of the received light of the transmitted light or the reflected light corresponding to the red light projected at the first light projection timing and a second pulse wave signal acquired according to a light intensity of the received light of the transmitted light or the reflected light corresponding to the infrared light projected at the second light projection timing to measure the oxygen saturation of the artery.

According to the ninth aspect, similarly to the first aspect, it is possible to implement an oxygen saturation measuring method capable of improving measurement accuracy.

An oxygen saturation measuring program according to a tenth aspect of the disclosure causes a processor to execute processing of: projecting, to an artery, reference light having a wavelength at which an absorption coefficient of oxidized hemoglobin and an absorption coefficient of reduced hemoglobin are higher than those of red light and infrared light; receiving, as received light, transmitted light or reflected light corresponding to the projected reference light; acquiring a third pulse wave signal corresponding to a light intensity of the received light of the reference light; based on a peak timing and a bottom timing of the acquired third pulse wave signal in one pulsation, determining a first light projection timing at which the red light is projected and a second light projection timing at which the infrared light is projected; projecting the red light at the determined first light projection timing; projecting the infrared light at the determined second light projection timing; and calculating oxygen saturation based on a first pulse wave signal acquired according to a light intensity of the received light of the transmitted light or the reflected light corresponding to the red light projected at the first light projection timing and a second pulse wave signal acquired according to a light intensity of the received light of the transmitted light or the reflected light corresponding to the infrared light projected at the second light projection timing to measure the oxygen saturation of the artery.

According to the tenth aspect, similarly to the first aspect, it is possible to implement an oxygen saturation measuring program capable of improving measurement accuracy. Advantageous Effects of Invention

With the oxygen saturation measuring device, the oxygen saturation measuring method, and the oxygen saturation measuring program according to the present disclosure, measurement accuracy can be improved.

BRIEF DESCRIPTION OF DRAWINGS

Fig. 1 is a perspective view showing an oxygen saturation measuring device according to an embodiment of the present disclosure.

Fig. 2 is a cross-sectional view for describing a sensor unit of the oxygen saturation measuring device according to the present embodiment.

Fig. 3 is a block diagram showing a hardware configuration of a processor of the oxygen saturation measuring device according to the embodiment.

Fig. 4 is a flowchart showing an oxygen saturation measuring method using the oxygen saturation measuring device according to the embodiment.

Fig. 5 is a timing chart for describing a state in which light projection timings of two rays of measurement light are determined according to a peak timing and a bottom timing of a third pulse wave signal of a reference light in the oxygen saturation measuring method using the oxygen saturation measuring device according to the embodiment.

Fig. 6 is a diagram for describing a method of setting each of the light projection timings of two rays of measurement light once based on the peak timing and the bottom timing of the third pulse wave signal of the reference light.

Fig. 7 is a diagram for describing a method of setting each of the light projection timings of two rays of measurement light a plurality of times based on the peak timing and the bottom timing of the third pulse wave signal of the reference light.

Fig. 8 is a diagram for describing a method of setting a first maximum side light projection period and a first minimum side light projection period for determining the light projection timings of two rays of measurement light based on a heartbeat interval obtained from the third pulse wave signal of the reference light.

Fig. 9 is a diagram for describing a method of setting the light projection timing of each of two rays of measurement light once in each of the first maximum side light projection period and the first minimum side light projection period.

Fig. 10A is a diagram for describing a method of setting a maximum side threshold value for determining the light projection timings of two rays of measurement light.

Fig. 10B is a diagram for describing a method of setting a minimum side threshold value for determining the light projection timings of two rays of measurement light.

Fig. 11 is a diagram for describing a method of setting the light projection timing of each of two rays of measurement light a plurality of times in each of the first maximum side light projection period and the first minimum side light projection period.

Fig. 12 is a graph for describing an amplitude change of a pulsation accompanying a body motion.

Fig. 13 is a graph for describing an amplitude change of a pulsation accompanying respiration.

Fig. 14A is a diagram for describing a waveform of a first pulse wave signal obtained from reflected light of red light projected to an artery to be measured.

Fig. 14B is a diagram for describing a waveform of a second pulse wave signal obtained from reflected light of infrared light projected to the artery to be measured.

Fig. 14C is a diagram for describing a waveform of a third pulse wave signal obtained from reflected light of reference light projected to the artery to be measured.

DESCRIPTION OF EMBODIMENTS

Hereinafter, the present embodiment will be described. In the following description of the drawings, the same reference numerals or similar reference numerals are assigned to the same portions and similar portions. However, the drawings are schematic, and a relationship between a thickness and plane dimensions, a ratio of a thickness of each apparatus or each member, and the like are different from actual ones. Therefore, specific thicknesses and dimensions should be determined in consideration of the following description. In addition, the drawings include portions having different dimensional relationships and ratios. Unless otherwise specified in the specification, the number of components according to the present disclosure is not limited to one, and a plurality of components may be present.

<Oxygen Saturation Measuring Device>

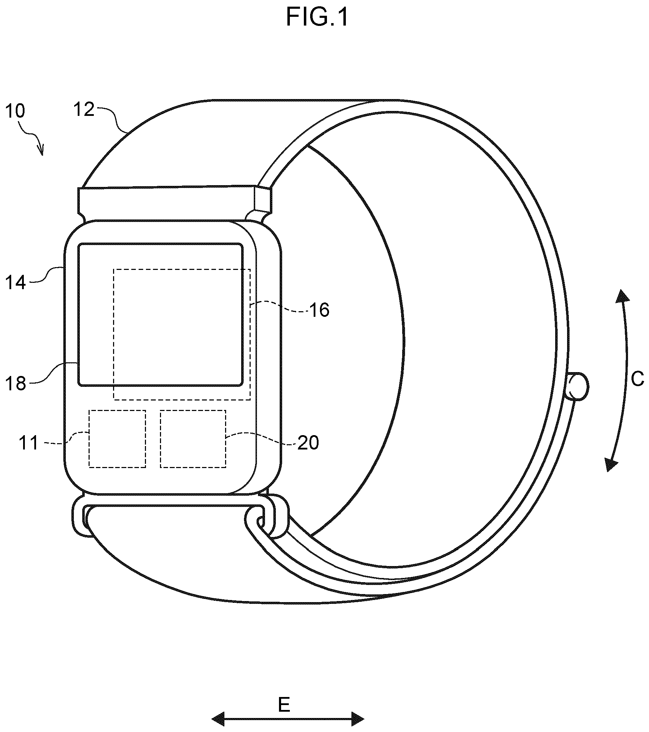

A structure of an oxygen saturation measuring device 10 according to the embodiment will be described with reference to Figs. 1 to 3. As shown in Fig. 1, the oxygen saturation measuring device 10 according to the embodiment is a portable wearable device that includes a band 12, a housing 14, a sensor unit 16, a display unit 18, and a computation control unit 20 and is wearable by a subject.

The oxygen saturation measuring device 10 according to the embodiment is provided with a drive power supply 11. The drive power supply 11 may be a primary battery or a secondary battery. In the disclosure, a shape of the oxygen saturation measuring device is not limited to a wearable device that is wearable by the subject. The oxygen saturation measuring device according to the disclosure may be a portable type that is wearable or unwearable, for example, a portable type capable of approaching a wrist, or may be arbitrarily configured like a stationary type or the like.

In the present specification, an "extending direction E of a forearm" overlaps with any of an extending direction of a radius of the subject, an extending direction of an ulna, and an extending direction of the artery. Strictly speaking, the "extending direction E of the forearm" is different for each subject. That is, the "extending direction E of the forearm" is not uniquely determined by coordinates in a three-dimensional space, but is individually determined based on the extending direction of the radius, the extending direction of the ulna, and the extending direction of the artery for each subject.

(Band)

As shown in Fig. 1, the band 12 is wound around the wrist of the subject in a circumferential direction C of the wrist. A material of the band 12 is any material such as resin, cloth, or metal. In addition, the band 12 is provided with a clasp for adjusting and fixing a length of the band 12 when the band 12 is wound around the wrist.

(Housing)

The housing 14 is attached to the band 12 and comes into contact with a surface on a dorsal side (that is, a back side of the hand) of the wrist of the subject. The housing 14 may be made of any material such as resin or metal. The housing 14 has a constant width in the extending direction E of the forearm. The sensor unit 16, the display unit 18, and the computation control unit 20 are provided in the housing 14.

(Display Unit)

As shown in Fig. 1, the display unit 18 is disposed on a surface of the housing 14 on a side opposite to the wrist. The display unit 18 is an image display device formed of, for example, liquid crystal or the like. The display unit 18 displays a computation result of the computation control unit 20 to the outside such that the subject can visually recognize the computation result. A storage device that temporarily stores a measurement result may be provided in the display unit 18.

(Sensor Unit)

The sensor unit 16 is attached to the band 12. The sensor unit 16 acquires a pulse wave signal of the artery of the wrist and measures oxygen saturation of the artery of the wrist based on the acquired pulse wave signal. The sensor unit according to the embodiment faces the artery included in a dorsal carpal arterial network, for example, on the dorsal side of the wrist of the subject. The dorsal carpal arterial network includes branches from a radial artery and branches from an ulnar artery. In the disclosure, the artery to be measured is not limited to the artery of the wrist.

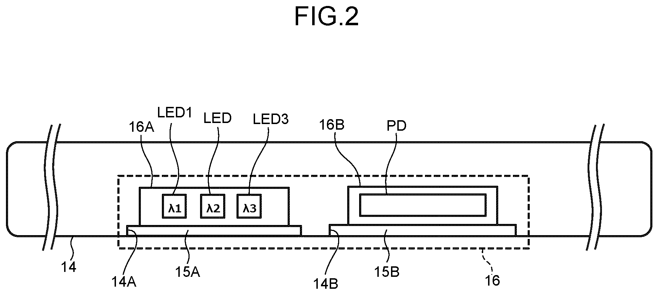

As shown in Fig. 2, the sensor unit 16 includes a light emitting unit 16A including a first light emitting element LED1, a second light emitting element LED2, and a third light emitting element LED3, and a light receiving unit 16B including a light receiving element PD. That is, in the embodiment, one "sensor unit" includes three light emitting elements and one light receiving element corresponding to the three light emitting elements. In the disclosure, a plurality of sensor units may be provided. The number of light emitting elements and the number of light receiving elements included in one "sensor unit" can be arbitrarily set.

In the housing 14, an opening 14A is formed between the light emitting unit 16A and the outside. An optical device 15A having translucency for light projected from the light emitting element is disposed in the opening 14A. The optical device 15A is, for example, a diffusion lens capable of enlarging an irradiation region. Although not shown, a diffusing agent may be disposed on an upper side of the light emitting element together with the diffusion lens or instead of the diffusion lens.

In the housing 14, an opening 14B is formed between the light receiving unit 16B and the outside. An optical device 15B having translucency for reflected light from the artery of the wrist is disposed in the opening 14B. The optical device 15B is, for example, a condenser lens capable of collecting the reflected light. In the disclosure, the optical device 15A and the optical device 15B are not essential.

(Light Emitting Element)

Each of the first light emitting element LED1, the second light emitting element LED2, and the third light emitting element LED3 is an electronic component such as a light emitting diode (LED). Each light emitting element irradiates the artery of the wrist with light. The first light emitting element LED1, the second light emitting element LED2, and the third light emitting element LED3 are disposed while being spaced apart from each other. In the disclosure, the number of each of first light emitting elements, second light emitting elements, and third light emitting elements is an arbitrary number such as one or more.

(First Light Emitting Element and Second Light Emitting Element)

The first light emitting element LED1 projects red light to the artery. The second light emitting element LED2 projects infrared light to the artery. In Fig. 2, a wavelength $\lambda 1$ of the red light is shown inside the first light emitting element LED1, and a wavelength $\lambda 2$ of the infrared light is shown inside the second light emitting element LED2. It is preferable that the first light emitting element LED1 and the second light emitting element LED2 are disposed such that optical paths of the light projected by the first light emitting element LED1 and the light projected by the second light emitting element LED2 are as close to each other as possible from the viewpoint of improving measurement accuracy.

(Red Light and Infrared Light)

In oxygen saturation measurement processing, it is necessary to use two wavelength bands having different absorption coefficients of oxidized hemoglobin and reduced hemoglobin in combination. As the combination of the wavelength bands, a combination of a red region having a wavelength band in a range of about 590 nm or more and 770 nm or less and an infrared region having a wavelength band in a range of about 770 nm or more and 1000 nm or less is preferable.

In particular, in order to measure the oxygen saturation with higher accuracy, it is desirable that a difference between the absorption coefficient of the oxidized hemoglobin and the absorption coefficient of the reduced hemoglobin is large. Therefore, a peak wavelength of the red light is preferably in a range of 640 nm or more and 660 nm or less, and a peak wavelength of the infrared light is preferably about 940 nm. In the embodiment, the red light and the infrared light are intermittently projected, for example, once in one pulsation of the artery.

(Third Light Emitting Element)

The third light emitting element LED3 projects reference light. The reference light has a wavelength having a higher signal-to-noise (SN) ratio than the red light and the infrared light with respect to the artery. In Fig. 2, a wavelength $\lambda 3$ of the reference light is shown inside the third light emitting element LED3. In the embodiment, the reference light is projected continuously, for example, 10 times or 20 times in one pulsation of the artery. In the disclosure, the number of times the reference light is projected can be arbitrarily set as long as a peak timing and a bottom timing can be calculated.

(Reference Light)

The reference light has a wavelength at which the absorption coefficient of the oxidized hemoglobin and the absorption coefficient of the reduced hemoglobin are higher than those of the red light and the infrared light. In the embodiment, an SN ratio of a pulse wave signal of received light corresponding to the reference light obtained by photoplethysmography (PPG) is higher than both the SN ratio of the red light and the SN ratio of the infrared light. As the reference light, for example, light in a wavelength band of 430 nm or more and 590 nm or less having a relatively large absorption coefficient in blood of the artery is preferable. Specifically, a blue region having a wavelength band of 430 nm or more and 490 nm or less and a green region having a wavelength band of 490 nm or more and 550 nm or less are preferable.

In the embodiment, in particular, green light having a peak wavelength of 530 nm or more and 540 nm or less is easily absorbed by hemoglobin in the blood, and a volume change of an arterial blood vessel is easily grasped, and thus is preferable from the viewpoint of obtaining the pulse wave signal having a relatively high SN ratio. In addition, an LED light source of a wavelength band of the green light has high availability in the market and is relatively inexpensive, and thus is advantageous as the third light emitting element that emits the reference light. In the disclosure, light sources of wavelength bands of a violet region and an ultraviolet region of 430 nm or less are also not excluded as light sources of the reference light.

In the disclosure, the reference light is not limited to the green light. In the disclosure, as long as the absorption coefficient of the oxidized hemoglobin and the absorption coefficient of the reduced hemoglobin are higher than those of the red light and the infrared light, light in any band that can be taken from a hemoglobin absorption spectrum can be adopted as the reference light.

(Light Receiving Element)

The light receiving element PD is an electronic component such as a photodiode (PD). The light receiving element PD is disposed at a position preset with respect to the light emitting element. In the embodiment, the number of light receiving elements PD is one, but in the disclosure, the number of light receiving elements may be plural.

The light receiving element PD receives the reflected light corresponding to the red light, and outputs a first pulse wave signal corresponding to a light intensity of the received reflected light. The light receiving element PD receives the reflected light corresponding to the infrared light, and outputs a second pulse wave signal corresponding to a light intensity of the received reflected light. In addition, the light receiving element PD receives the reflected light corresponding to the reference light, and outputs a third pulse wave signal corresponding to a light intensity of the received reflected light. For specific oxygen saturation measurement, two types of reflected light of the red light and the infrared light received by the light receiving element are used.

In the disclosure, the light receiving element is not limited to the reflected light of each of the red light, the infrared light, and the reference light, and may receive transmitted light corresponding to each light and output each pulse wave signal corresponding to a light intensity of the received transmitted light. That is, although the oxygen saturation measuring device 10 according to the embodiment is a reflection type in which an intensity of the pulse wave signal is measured by the reflected light, the disclosure is not limited to the reflection type, and a transmission type oxygen saturation measuring device in which the intensity of the pulse wave signal is measured by the transmitted light may be configured.

Although not shown, a light blocking portion may be provided between the light emitting element and the light receiving element. The light blocking portion prevents the light receiving element from directly receiving the light from the light emitting element.

(Oxygen Saturation Measurement Principle)

Here, an oxygen saturation measurement principle using two types of irradiation light and two types of reflected light will be described. Specifically, a change in amplitude of the pulse wave signal of the reflected light of the red light is monitored over time, and a fluctuation component (in other words, AC) of the pulse wave signal included in the monitored pulse wave signal and a fixed component (in other words, DC) that does not fluctuate are calculated. Then, by dividing the fluctuation component by the fixed component (AC/DC), a perfusion index (PI) value of the red light is calculated as $PI_{RED}$. In addition, similarly to the red light, a PI value ($PI_{IR}$) of reflected light of near-infrared light is calculated by monitoring a change in intensity of the pulse wave signal of the reflected light of the near-infrared light over time.

Then, a ratio ($PI_{RED}/PI_{IR}$) between the PI value of the red light ($PI_{RED}$) and the PI value of the near-infrared light ($PI_{IR}$) is calculated. Then, the oxygen saturation can be calculated by using the calculated ratio ($PI_{RED}/PI_{IR}$) in the following Equation (1).

$$\text{Oxygen Saturation } [\%] = a \times (PI_{RED}/PI_{IR}) + b \cdots \text{Equation (1)}$$

Coefficients a and b in Equation (1) can be obtained by experiments.

(Computation Control Unit)

In the embodiment, the computation control unit 20 is provided in the housing 14. A processor is electrically connected to the first light emitting element, the second light emitting element, the third light emitting element, and the light receiving element PD. Data of the pulse wave signal of the reflected light corresponding to the light projected from each light emitting element is input from the light receiving element PD to the computation control unit 20 over time. The computation control unit 20 measures the oxygen saturation of the artery irradiated with light by a method using the ratio of the PI values or the like based on the pulse wave signal acquired from the reflected light.

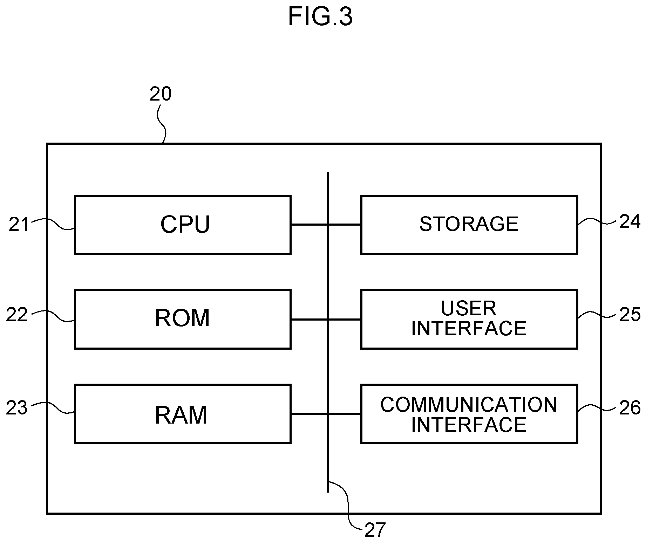

As shown in Fig. 3, the computation control unit 20 includes a central processing unit (CPU) 21 (processor), a read only memory (ROM) 22, a random access memory (RAM) 23, a storage 24, a user interface 25, and a communication interface 26. The respective components are communicably connected to each other via a bus 27.

The CPU 21 is a central processing unit, executes various programs, and controls each unit. That is, the CPU 21 reads a program from the ROM 22 or the storage 24, and executes the program by using the RAM 23 as a work area. The CPU 21 performs control of each component described above and executes various types of computation processing according to a program recorded in the ROM 22 or the storage 24. The CPU 21 is the processor according to the disclosure.

In the embodiment, an oxygen saturation measuring program is stored in the ROM 22 or the storage 24. The oxygen saturation measuring program is a computation program for measuring the oxygen saturation.

The ROM 22 stores various programs and various data. The RAM 23 functions as a work area and temporarily stores a program or data. The storage 24 is implemented by a hard disk drive (HDD) or a solid state drive (SSD), and stores various programs including an operating system and various data.

The user interface 25 is an interface when the subject wearing the oxygen saturation measuring device 10 that is a wearable device uses the computation control unit 20. The user interface 25 can include, for example, at least one of a liquid crystal display including a touch panel that enables a touch operation by the subject, a voice input receiving unit that receives a voice input from the subject, a button that can be pressed by the subject, and the like. The display unit according to the embodiment is an example of the user interface 25.

The communication interface 26 is an interface for the computation control unit 20 to communicate with other devices, and for example, standards such as Ethernet (registered trademark), FDDI, and Wi-Fi (registered trademark) are used.

When executing the oxygen saturation measuring program, the oxygen saturation measuring device 10 implements various functions using the above hardware resources. The oxygen saturation measuring device 10 includes a light projection timing determination unit, a light emission control unit, and an oxygen saturation measurement unit as functional configurations implemented by the oxygen saturation measuring device 10. Each functional configuration is implemented by the CPU 21 reading and executing the oxygen saturation measuring program stored in the ROM 22 or the storage 24.

<Oxygen Saturation Measuring Method>

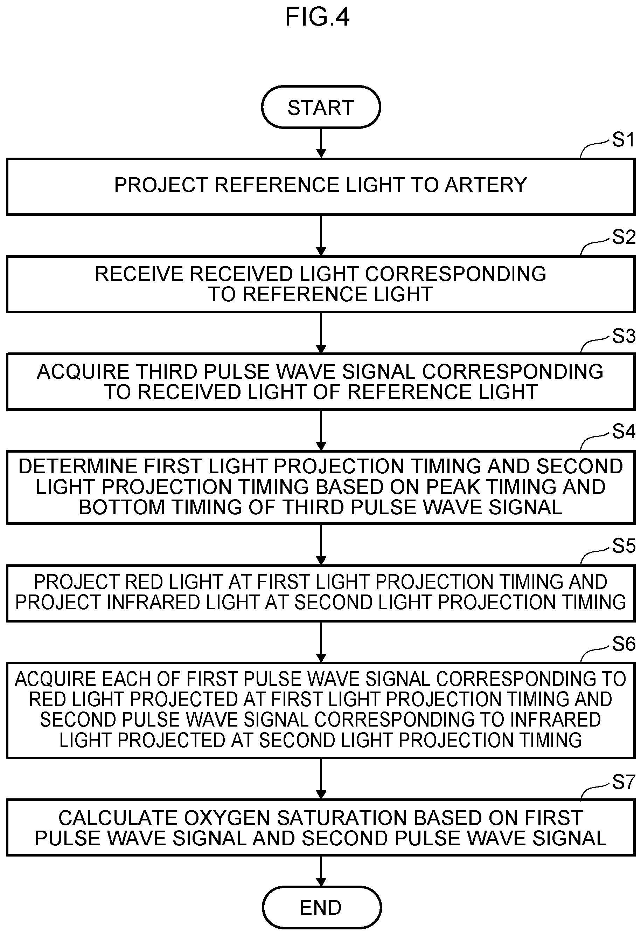

Next, an example of an oxygen saturation measuring method using the oxygen saturation measuring device 10 according to the embodiment will be described with reference to Figs. 4 to 13.

First, as shown in step S1 in Fig. 4, the processor 21 projects the reference light to the artery to be measured by the third light emitting element LED3.

Next, as shown in step S2 in Fig. 4, the processor 21 receives the received light corresponding to the reference light by the light receiving element PD. The light receiving element PD outputs a third pulse wave signal PS3 corresponding to the received light of the reference light to the processor 21. As a result, as shown in step S3 in Fig. 4, the processor 21 acquires the third pulse wave signal PS3.

(Light Projection Timing Determination Processing)

Next, as shown in step S4 in Fig. 4, the processor 21 determines a first light projection timing and a second light projection timing based on a peak timing and a bottom timing of the third pulse wave signal PS3.

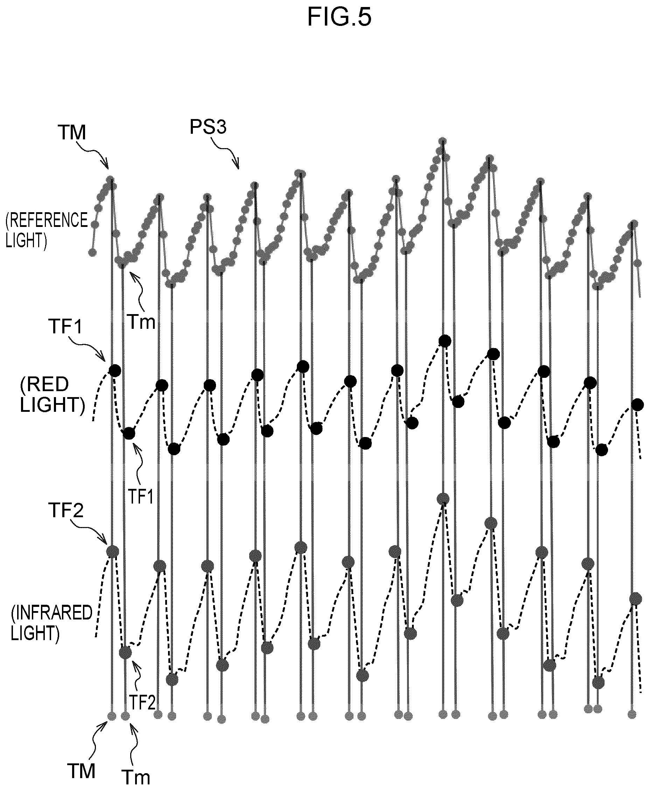

Specifically, as shown in Fig. 5, the light projection timing determination unit of the processor 21 calculates a peak timing TM and a bottom timing Tm of the third pulse wave signal PS3 in one pulsation. Then, the processor 21 determines the first light projection timing at which the first light emitting element LED1 projects the red light and the second light projection timing at which the second light emitting element LED2 projects the infrared light based on the calculated peak timing TM and bottom timing Tm.

(Light Emission Control Processing)

Next, as shown in step S5 in Fig. 4, the processor 21 projects the red light at the first light projection timing and projects the infrared light at the second light projection timing. Specifically, the light emission control unit of the processor 21 controls the first light emitting element LED1 and the second light emitting element LED2.

The light emission control unit causes the first light emitting element LED1 to project the red light at a first light projection timing TF1, and causes the second light emitting element LED2 to project the red light at a second light projection timing TF2. In the uppermost part of Fig. 5, a locus of a data point of the third pulse wave signal PS3 of the received light corresponding to the reference light and virtual lines extending vertically downward from respective positions of the peak timing TM and the bottom timing Tm extracted from the locus are shown.

The peak timing TM of the reference light in the uppermost part of Fig. 5, the first light projection timing TF1 of the red light in the middle part of Fig. 5, and the second light projection timing TF2 of the infrared light in the lowermost part of Fig. 5 are positioned on the same virtual line, thereby indicating that the peak timing TM of the reference light, the first light projection timing TF1 of the red light, and the second light projection timing TF2 of the infrared light are the same time.

In addition, the bottom timing Tm of the reference light in the uppermost part of Fig. 5, the first light projection timing TF1 of the red light in the middle part of Fig. 5, and the second light projection timing TF2 of the infrared light in the lowermost part of Fig. 5 are positioned on the same virtual line, thereby indicating that the bottom timing Tm of the reference light, the first light projection timing TF1 of the red light, and the second light projection timing TF2 of the infrared light are the same time.

That is, in a projection pattern shown in Fig. 5, the red light and the infrared light are projected in synchronization with the peak timing TM of the reference light and in synchronization with the bottom timing Tm. The light receiving element PD outputs the first pulse wave signal PS1 according to the red light projected at the first light projection timing TF1. In addition, the light receiving element PD outputs the second pulse wave signal PS2 according to the infrared light projected at the second light projection timing TF2.

(Oxygen Saturation Measurement Processing)

Next, as shown in step S6 in Fig. 4, the processor 21 acquires the first pulse wave signal PS1 corresponding to the red light projected at the first light projection timing TF1 and the second pulse wave signal PS2 corresponding to the infrared light projected at the second light projection timing TF2. Then, as shown in step S7 in Fig. 4, the oxygen saturation measurement unit of the processor 21 calculates the oxygen saturation of the artery based on the first pulse wave signal PS1 and the second pulse wave signal PS2 output from the light receiving element PD. The calculated oxygen saturation is displayed on the display unit 18 as a measured value.

(Specific Example of Light Projection Timing Determination Processing)

Next, as a specific example of light projection timing determination processing described above, processing 1 and processing 2 in the embodiment will be described in detail. processing 1 is a method mainly using a heartbeat interval of the third pulse wave signal PS3. processing 2 is a method mainly using an amplitude of the third pulse wave signal PS3.

(Processing 1)

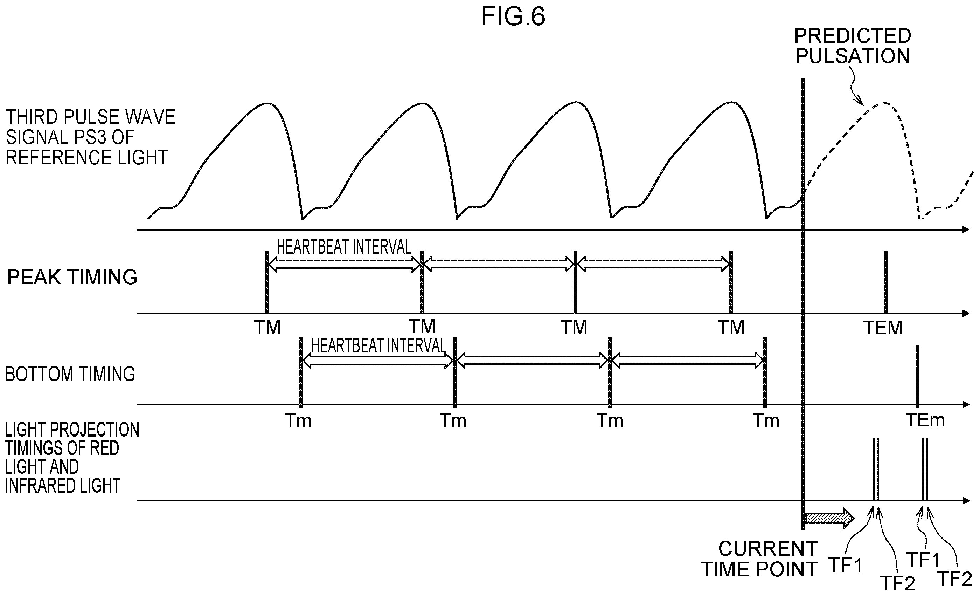

First, processing 1 will be described with reference to Figs. 6 to 8. As shown in Fig. 6, the processor 21 calculates the heartbeat interval of a predicted pulsation of the third pulse wave signal PS3 expected to arrive in the future based on the peak timing TM and the bottom timing Tm of the third pulse wave signal PS3 actually acquired in the past. In the uppermost part of Fig. 6, the third pulse wave signal PS3 of the reference light is shown. The heartbeat interval of the predicted pulsation can be calculated, for example, as an average of heartbeat intervals of a plurality of actually acquired third pulse wave signals PS3. In the disclosure, a method of calculating the heartbeat interval of the predicted pulsation is not limited to the average, and any method can be adopted.

In addition, the processor 21 calculates a predicted peak timing TEM and a predicted bottom timing TEm in the predicted pulsation. The second row from the top in Fig. 6 shows the peak timing TM and the predicted peak timing TEM of the reference light, and the third row from the top in Fig. 6 shows the bottom timing Tm and the predicted bottom timing TEm of the reference light.

The predicted peak timing TEM can be calculated, for example, by adding the heartbeat interval of the predicted pulsation to the peak timing TM acquired at a current time point. Similarly, the predicted bottom timing TEm can be calculated, for example, by adding the heartbeat interval of the predicted pulsation to the bottom timing Tm acquired most recently at the current time point. In the disclosure, a method of calculating the predicted peak timing TEM and the predicted bottom timing TEm is not limited thereto, and any method can be adopted.

Then, in the embodiment, the processor 21 is configured to determine the first light projection timing TF1 and the second light projection timing TF2 in the predicted pulsation based on the calculated predicted peak timing TEM and predicted bottom timing TEm. In the lowermost part of Fig. 6, a case in which the first light projection timing TF1 of the red light and the second light projection timing TF2 of the infrared light are set once based on the peak timing TM and the bottom timing Tm, respectively, is shown.

In addition, in a light projection pattern in Fig. 6, the first light projection timing TF1 and the second light projection timing TF2 calculated according to a maximum amplitude of the predicted pulsation are earlier than the peak timing TM. In addition, in the light projection pattern in Fig. 6, the first light projection timing TF1 and the second light projection timing TF2 calculated according to a minimum amplitude of the predicted pulsation are later than the bottom timing Tm. As shown in Fig. 6, the first light projection timing TF1 and the second light projection timing TF2 do not need to be strictly synchronized with the peak timing TM and the bottom timing Tm, and may be determined based on the peak timing TM and the bottom timing Tm.

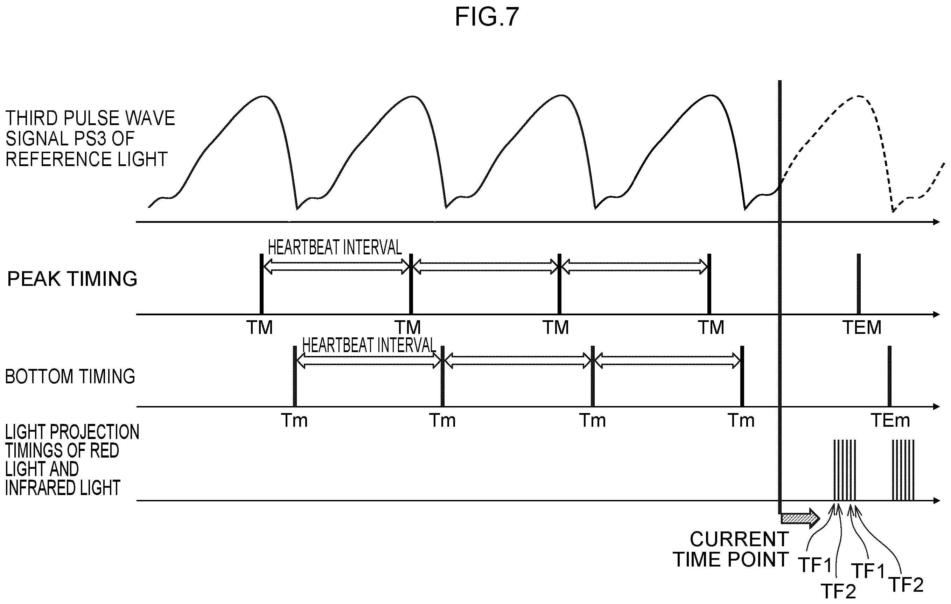

Furthermore, as shown in Fig. 7, a plurality of first light projection timings TF1 and a plurality of second light projection timings TF2 may be determined based on the peak timing TM and the bottom timing Tm, respectively.

(First Maximum Side Light Projection Period and First Minimum Side Light Projection Period)

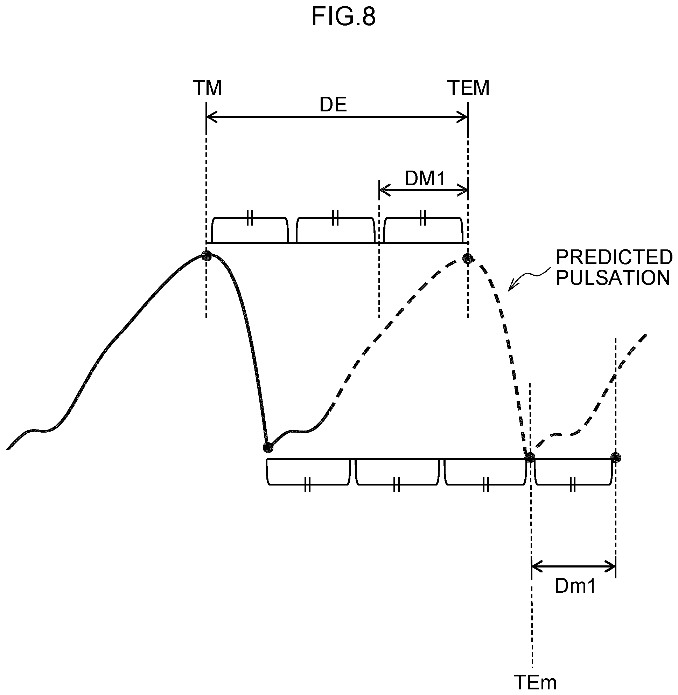

As shown in Fig. 8, in processing 1, the processor 21 can further set a first maximum side light projection period DM1 and a first minimum side light projection period Dm1.

Specifically, the processor 21 sets, as the first maximum side light projection period DM1, a period from a timing at which 2/3 of a heartbeat interval DE of the predicted pulsation elapses from the peak timing TM in a pulsation immediately before the predicted pulsation to the predicted peak timing TEM.

In addition, the processor 21 sets, as the first minimum side light projection period Dm1, a period from the predicted bottom timing TEm of the predicted pulsation to a timing at which 1/3 of the heartbeat interval DE of the predicted pulsation elapses from the predicted bottom timing TEm. Then, the processor 21 is configured to determine the first light projection timing TF1 and the second light projection timing TF2 in each of the set first maximum side light projection period DM1 and first minimum side light projection period Dm1.

In a case in which the first light projection timing TF1 and the second light projection timing TF2 are earlier than the timing at which 2/3 of the heartbeat interval DE elapses or are timings after the predicted peak timing TEM, a value obtained as the maximum amplitude is excessively different from the actual maximum amplitude of the artery, and thus the measurement accuracy is deteriorated. In addition, in a case in which the first light projection timing TF1 and the second light projection timing TF2 are earlier than the predicted bottom timing TEm or are timings after the timing at which 1/3 of the heartbeat interval DE elapses, a value obtained as the minimum amplitude is excessively different from the actual minimum amplitude of the artery, and thus the measurement accuracy is deteriorated.

(Processing 2)

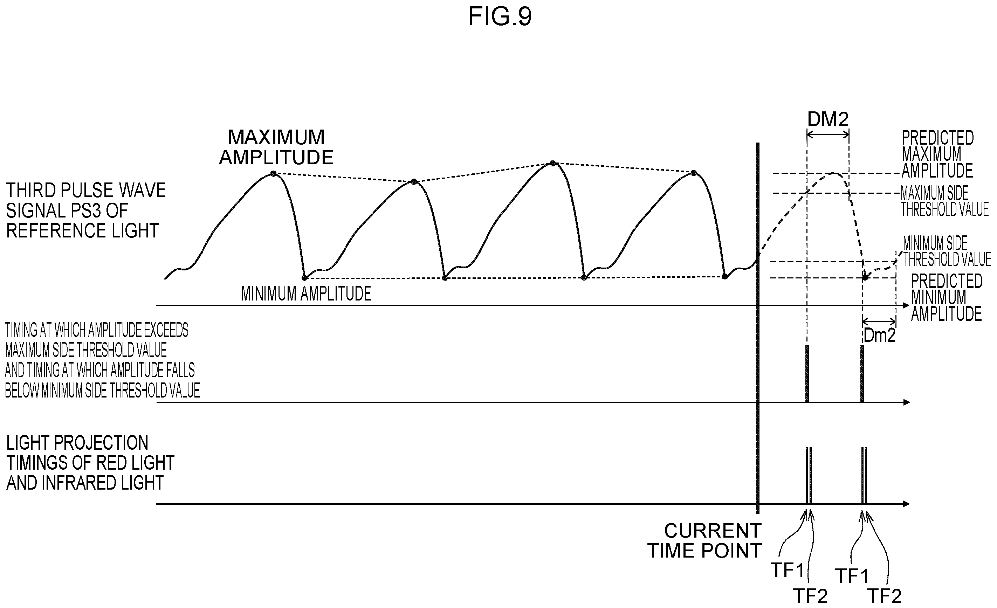

Next, processing 2 will be described with reference to Figs. 9 to 11.

(Second Maximum Side Light Projection Period and Second Minimum Side Light Projection Period)

As shown in Fig. 9, the processor 21 sets in advance a predicted maximum amplitude in the predicted pulsation of the third pulse wave signal PS3 and a maximum side threshold value of the amplitude based on a maximum amplitude of the third pulse wave signal PS3 actually acquired in the past at the peak timing TM. The predicted maximum amplitude and the maximum side threshold value are calculated to determine the first light projection timing TF1 and the second light projection timing TF2.

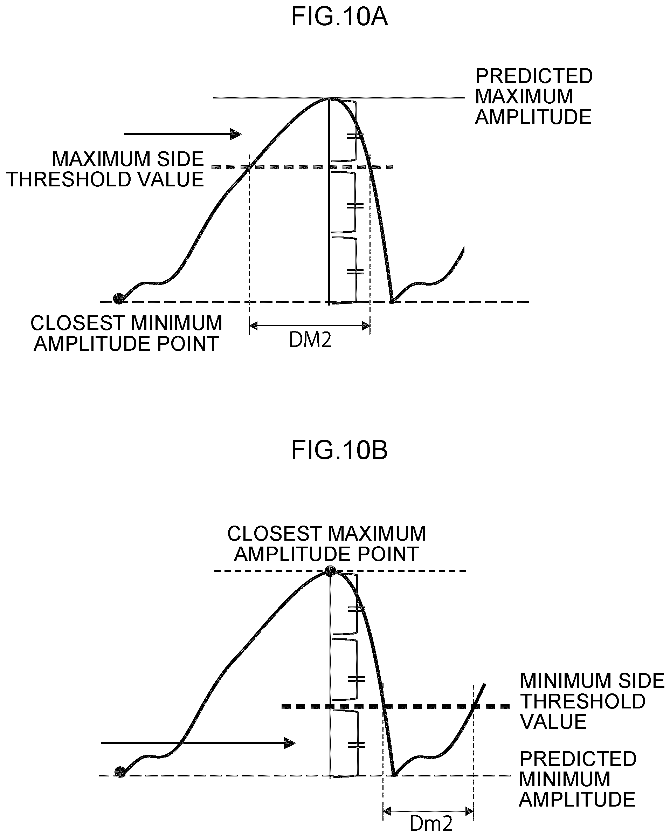

The predicted maximum amplitude can be calculated by, for example, an average of maximum amplitudes acquired in the past. In the disclosure, a method of calculating the predicted maximum amplitude is not limited to the average, and any method can be adopted. As shown in Fig. 10 A, the maximum side threshold value can be set to 2/3 or more of a difference between the maximum amplitude and the minimum amplitude of the third pulse wave signal PS3, for example. In a case in which the maximum side threshold value is less than 2/3 of the difference between the maximum amplitude and the minimum amplitude, the value obtained as the maximum amplitude is excessively different from the actual maximum amplitude of the artery, and thus, the measurement accuracy is deteriorated.

Therefore, the processor 21 is configured to be able to determine the first light projection timing TF1 and the second light projection timing TF2 when the continuously acquired amplitude of the third pulse wave signal PS3 rises to the maximum side threshold value or higher. In other words, the processor 21 is configured to be unable to determine the first light projection timing TF1 and the second light projection timing TF2 when the changing amplitude of the third pulse wave signal PS3 starts to fall beyond the maximum threshold value and then becomes lower than the maximum side threshold value. As a result, a second maximum side light projection period DM2 in which the amplitude is equal to or higher than the maximum side threshold value is formed in the actual pulsation of the third pulse wave signal PS3.

The processor 21 sets in advance a predicted minimum amplitude in the predicted pulsation of the third pulse wave signal PS3 and a minimum side threshold value of the amplitude based on the minimum amplitude of the third pulse wave signal PS3 actually acquired in the past at the bottom timing Tm. The predicted minimum amplitude and the minimum side threshold value are calculated to determine the first light projection timing TF1 and the second light projection timing TF2.

The predicted minimum amplitude can be calculated by, for example, an average of minimum amplitudes acquired in the past. In the disclosure, a method of calculating the predicted minimum amplitude is not limited to the average, and any method can be adopted. As shown in Fig. 10B, the minimum side threshold value can be set to, for example, 1/3 or less of the difference between the maximum amplitude and the minimum amplitude of the third pulse wave signal PS3. In a case in which the minimum side threshold value exceeds 1/3 of the difference between the maximum amplitude and the minimum amplitude, the value obtained as the minimum amplitude is excessively different from the actual minimum amplitude of the artery, and thus, the measurement accuracy is deteriorated.

Therefore, the processor 21 is configured to be able to determine the first light projection timing TF1 and the second light projection timing TF2 when the continuously acquired amplitude of the third pulse wave signal PS3 falls to the minimum side threshold value or lower. In other words, the processor 21 is configured to be unable to determine the first light projection timing TF1 and the second light projection timing TF2 when the changing amplitude of the third pulse wave signal PS3 starts to rise beyond the minimum threshold value and then exceeds the minimum side threshold value. As a result, a second minimum side light projection period Dm2 in which the amplitude is equal to or lower than the minimum side threshold value is formed in the actual pulsation of the third pulse wave signal PS3.

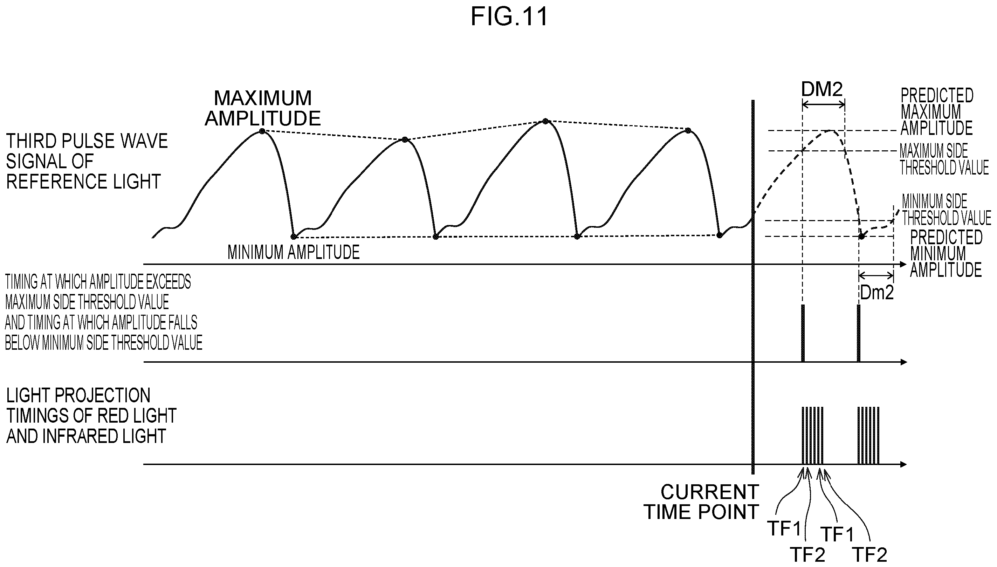

As shown in Fig. 11, a plurality of first light projection timings TF1 and a plurality of second light projection timings TF2 may be determined in each of the second maximum side light projection period DM2 and the second minimum side light projection period Dm2.

(Switching between processing 1 and processing 2)

In the embodiment, processing 1 and processing 2 may be switched based on a coefficient of variation indicating a state of the pulsation of the subject. Specifically, the processor 21 calculates the coefficient of variation based on the heartbeat interval of the third pulse wave signal PS3 actually acquired in the past. The coefficient of variation can be calculated, for example, by dividing a standard deviation of the heartbeat intervals at a constant pulse rate such as 10 beats acquired in the past by an average value of the heartbeat intervals at the constant pulse rate. In the disclosure, the number of beats used for the calculation of the coefficient of variation is not limited to 10 beats.

Then, in a case in which the calculated coefficient of variation is equal to or less than a preset threshold value, the processor 21 executes (processing 1) which is a determination method based on the heartbeat interval. On the other hand, in a case in which the coefficient of variation exceeds the threshold value, the processor 21 executes (processing 2) which is a determination method based on the amplitude.

(Threshold value)

In the embodiment, the threshold value of the coefficient of variation is 0.1 or less. The disclosure is not limited thereto, and the threshold value can be changed if appropriate.

Here, for example, in a case in which a variation in the heartbeat interval such as an irregular heartbeat occurs, there is a possibility that the measurement accuracy is deteriorated in the determination method of (processing 1) based on the heartbeat interval. For this reason, in the embodiment, the threshold value is set to determine whether or not the variation in the heartbeat interval occurs.

In a case in which it is determined that the variation in the heartbeat interval is likely to occur due to the coefficient of variation exceeding the threshold value, (processing 2) based on the amplitude is executed instead of (processing 1). Therefore, the measurement accuracy for the oxygen saturation can be improved.

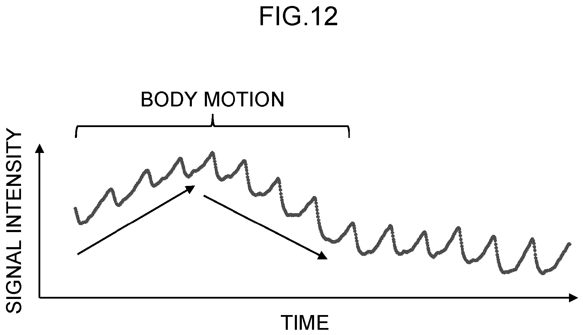

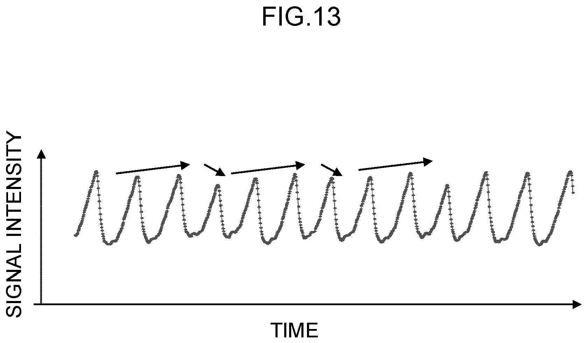

On the other hand, in the case of the determination method of (processing 2) based on the amplitude, when an amplitude change occurs due to respiration, a body motion, or the like of the subject, there is a possibility that the measurement accuracy for the oxygen saturation is deteriorated. In Fig. 12, a pulse wave signal in which a baseline variation is indicated by a plurality of arrows is shown as an example of an amplitude change accompanying the body motion. In Fig. 13, a pulse wave signal in which a baseline variation is indicated by a plurality of arrows is shown as an example of an amplitude change accompanying the respiration.

Therefore, in the embodiment, in a case in which it is determined that the variation in the heartbeat interval is less likely to occur due to the coefficient of variation being equal to or lower than the threshold value, (processing 1) based on the heartbeat interval is executed instead of (processing 2). As a result, it is possible to suppress an influence of the amplitude change caused by the respiration, the body motion, or the like on the oxygen saturation measurement.

(Comparative Example)

On the other hand, in a comparative example in which only the red light and the infrared light are projected as in the embodiment, the maximum amplitude and the minimum amplitude of each of the red light and the infrared light are obtained, and thus, the number of times each of the red light and the infrared light is projected is increased. Therefore, input power for light projection increases. Therefore, in the comparative example, it is necessary to repeat the projection of the red light and the infrared light several tens of times in one pulsation of the artery, for example, at an interval shorter than the heartbeat interval.

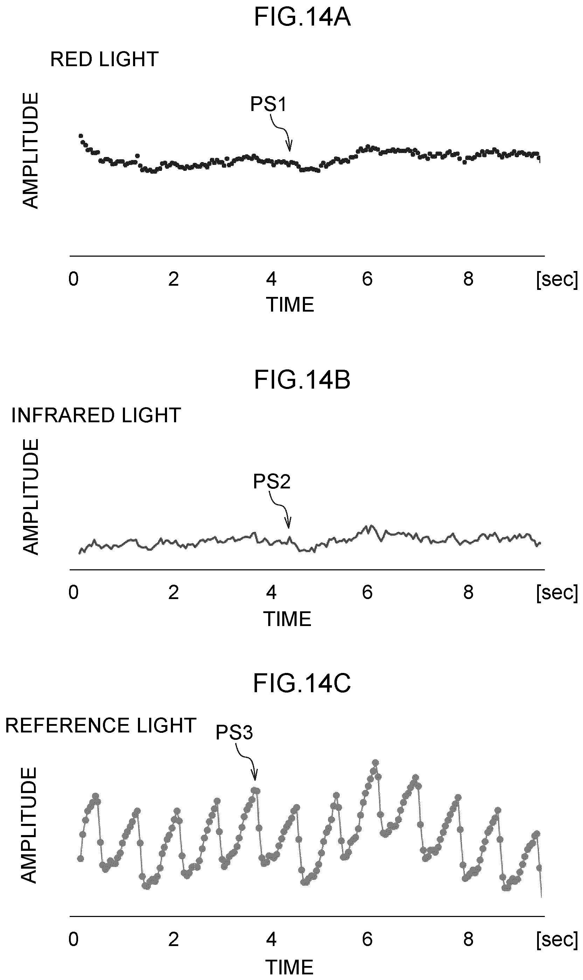

In addition, a waveform of the pulse wave signal corresponding to each of the red light shown in Fig. 14B and the infrared light shown in Fig. 14 C is unclear as compared with a waveform of the pulse wave signal corresponding to the reference light shown in Fig. 14A. For this reason, the degree of calculation of the amplitude decreases, and as a result, the measurement accuracy for the oxygen saturation is deteriorated.

(Actions and Effects)

In the embodiment, the wavelength of the reference light projected to the artery is a wavelength at which the absorption coefficient of the oxidized hemoglobin and the absorption coefficient of the reduced hemoglobin are higher than those of the red light and the infrared light. Therefore, the SN ratio of the third pulse wave signal PS3 corresponding to the reference light is higher than those of the first pulse wave signal PS1 corresponding to the red light and the second pulse wave signal PS2 corresponding to the infrared light. In addition, the first light projection timing TF1 for projecting the red light and the second light projection timing TF2 for projecting the infrared light are determined based on the peak timing TM and the bottom timing Tm in one pulsation of the third pulse wave signal PS3.

Then, the oxygen saturation of the artery is measured based on the first pulse wave signal PS1 output from the light receiving element PD according to the red light projected at the first light projection timing TF1 and the second pulse wave signal PS2 output from the light receiving element PD according to the infrared light projected at the second light projection timing TF2.

In the waveform of the third pulse wave signal PS3 in one pulsation, the maximum amplitude that is a peak value on a maximum side of the amplitude is obtained at the peak timing TM, and the minimum amplitude that is a peak value on a minimum side of the amplitude is obtained at the bottom timing Tm. Therefore, by projecting the red light for measurement at the first light projection timing TF1 determined according to the peak timing TM and the bottom timing Tm, the calculation accuracy for the amplitude of the first pulse wave signal PS1 of the received light corresponding to the red light is improved.

Similarly, since the infrared light for measurement is projected at the second light projection timing TF2 determined according to the peak timing TM and the bottom timing Tm, the calculation accuracy of the amplitude of the second pulse wave signal PS2 of the received light corresponding to the infrared light is improved. As a result, the measurement accuracy for the oxygen saturation measured based on the first pulse wave signal PS1 and the second pulse wave signal PS2 is improved.

In addition, in the embodiment, the first light projection timing TF1 and the second light projection timing TF2 are determined according to the peak timing TM and the bottom timing Tm formed once each in one pulsation. That is, the number of times of light projection is performed is at least twice according to one peak timing TM and one bottom timing Tm. Therefore, it is possible to reduce the input power as compared with a case in which the projection of each of the red light and the infrared light is repeated a plurality of times, for example, 50 times in one pulsation of the artery to be measured at an interval shorter than the heartbeat interval. In addition, the number of times each of the red light and the infrared light is projected and the number of times each of the red light and the infrared light is received can be reduced.

In addition, in the embodiment, the measuring device can have a long life as the input power for the projection of each of the red light and the infrared light is reduced. In addition, in a case in which the saved input power is redistributed as projection power for the red light and projection power for the infrared light within a range in which a certain measurement accuracy is ensured, the SN ratio of the red light and the SN ratio of the infrared light can be improved. As a result, the measurement accuracy for the oxygen saturation can be further improved.

In the embodiment, the predicted peak timing TEM and the predicted bottom timing TEm in the predicted pulsation are calculated based on the peak timing TM and the bottom timing Tm of the actually acquired third pulse wave signal PS3.

The first light projection timing TF1 and the second light projection timing TF2 in the predicted pulsation are determined based on the calculated predicted peak timing TEM and predicted bottom timing TEm. Therefore, the first light projection timing TF1 and the second light projection timing TF2 can be determined with high accuracy.

In addition, in the embodiment, the period from the timing at which 2/3 of the heartbeat interval DE of the predicted pulsation elapses from the peak timing TM in a pulsation immediately before the predicted pulsation to the predicted peak timing TEM is set as the first maximum side light projection period DM1. In addition, the period from the predicted bottom timing TEm of the predicted pulsation to the timing at which 1/3 of the heartbeat interval DE of the predicted pulsation elapses from the predicted bottom timing TEm is set as the first minimum side light projection period Dm1. In addition, the first light projection timing TF1 and the second light projection timing TF2 are determined in each of the set first maximum side light projection period DM1 and first minimum side light projection period Dm1. Therefore, the first light projection timing TF1 and the second light projection timing TF2 can be determined with higher accuracy.

In addition, in the embodiment, the maximum side threshold value of the amplitude for determining the first light projection timing TF1 and the second light projection timing TF2 is set based on the maximum amplitude of the third pulse wave signal PS3 actually acquired at the peak timing. The second maximum side light projection period DM2 in which the amplitude of the third pulse wave signal PS3 in the predicted pulsation is equal to or higher than the maximum side threshold value is set.

In addition, the minimum side threshold value of the amplitude for determining the first light projection timing TF1 and the second light projection timing TF2 is set based on the minimum amplitude of the third pulse wave signal PS3 actually acquired at the bottom timing. The second minimum side light projection period in which the amplitude of the third pulse wave signal PS3 in the predicted pulsation is equal to or higher than the minimum side threshold value is set.

Then, the first light projection timing TF1 and the second light projection timing TF2 are determined in each of the set second maximum side light projection period DM2 and second minimum side light projection period. Therefore, the first light projection timing TF1 and the second light projection timing TF2 can be determined with high accuracy.

In the embodiment, the maximum side threshold value is 2/3 or more of the difference between the maximum amplitude and the minimum amplitude of the third pulse wave signal PS3, and the minimum side threshold value is 1/3 or less of the difference between the maximum amplitude and the minimum amplitude of the third pulse wave signal PS3. Therefore, the first light projection timing TF1 and the second light projection timing TF2 can be determined with higher accuracy.

In addition, in the embodiment, the coefficient of variation representing the state of the pulsation of the subject is calculated based on the heartbeat interval of the actually acquired third pulse wave signal PS3. In addition, in a case in which the calculated coefficient of variation is equal to or less than the preset threshold value, the processor 21 executes (processing 1) to determine the first light projection timing TF1 and the second light projection timing TF2 in each of the first maximum side light projection period DM1 and the first minimum side light projection period Dm1.

In a case in which the coefficient of variation exceeds the threshold value, the processor 21 executes (processing 2) to determine the first light projection timing TF1 and the second light projection timing TF2 in each of the second maximum side light projection period DM2 and the second minimum side light projection period. That is, a method of determining the first light projection timing TF1 and the second light projection timing TF2 is switched according to the state of the pulsation of the subject. Therefore, preferable oxygen saturation measurement processing can be selected according to the state of the pulsation of the subject.

In the embodiment, the threshold value of the coefficient of variation is set to 0.1 or less. Therefore, switching accuracy of the oxygen saturation measurement processing can be improved.

In addition, in the case of a measurement device such as a wearable device driven by a relatively compact primary battery or secondary battery, it is difficult to increase light projection power per one time because it is necessary to avoid shortening of a continuous use time. However, in the embodiment, a calculation load is reduced, and thus, the input power can be reduced. Therefore, it is possible to implement a wearable device capable of achieving a long life.

<Other Embodiments>

Although the disclosure has been described with reference to the above disclosed embodiments, it should not be understood that the description and drawings constituting a part of the disclosure limit the disclosure.

For example, in the disclosure, the oxygen saturation measurement processing executed by the CPU 21 reading software (program) in the above-described embodiment may be executed by various processors other than the CPU. Examples of the processor in this case include a programmable logic device (PLD) in which a circuit configuration can be changed after manufacturing a field-programmable gate array (FPGA) or the like, a dedicated electric circuit that is a processor having a circuit configuration exclusively designed for executing specific processing such as an application specific integrated circuit (ASIC).

In addition, the oxygen saturation measurement processing may be executed by one of these various processors, or may be executed by a combination of two or more processors of the same type or different types (for example, a plurality of FPGAs or a combination of a CPU and an FPGA). More specifically, a hardware structure of these various processors is an electric circuit in which circuit elements such as semiconductor elements are combined.

In each embodiment described above, an aspect in which the oxygen saturation measuring program is stored (installed) in advance in the ROM 22 or the storage 24 has been described, but the disclosure is not limited thereto. The program may be provided in a form of being recorded in a recording medium such as a compact disk read only memory (CD-ROM), a digital versatile disk read only memory (DVD-ROM), or a universal serial bus (USB) memory. In addition, the program may be downloaded from an external apparatus via a network.

The disclosure includes various embodiments and the like that are not described above, and the technical scope of the disclosure is defined only by appropriate specific matters of the invention in the claims from the above description.

The disclosure of Japanese Patent Application No. 2022-167840 filed on October 19, 2022 is incorporated herein by reference in its entirety.

All documents, patent applications, and technical standards mentioned herein are incorporated herein by reference to the same extent as if each individual document, patent application, and technical standard were specifically and individually stated.

**Claims**

**1.** An oxygen saturation measuring device, comprising:

a sensor unit including a first light emitting element that projects red light to an artery, a second light emitting element that projects infrared light to the artery, a third light emitting element that projects reference light, which has a wavelength at which an absorption coefficient of oxidized hemoglobin and an absorption coefficient of reduced hemoglobin are higher than those of red light and infrared light, to the artery, and a light receiving element that receives, as received light, transmitted light or reflected light corresponding to each of the projected red light, the projected infrared light, and the projected reference light, and outputs a first pulse wave signal corresponding to a light intensity of the received light of the red light, a second pulse wave signal corresponding to a light intensity of the received light of the infrared light, and a third pulse wave signal corresponding to a light intensity of the received light of the reference light; and

a processor electrically connected to the first light emitting element, the second light emitting element, and the light receiving element, and configured to determine, based on a peak timing and a bottom timing of the third pulse wave signal in one pulsation, a first light projection timing at which the first light emitting element projects the red light and a second light projection timing at which the second light emitting element projects the infrared light, cause the first light emitting element to project the red light at the determined first light projection timing, cause the second light emitting element to project the infrared light at the determined second light projection timing, and calculate oxygen saturation based on the first pulse wave signal acquired according to the light intensity of the received light of the red light projected at the first light projection timing and the second pulse wave signal acquired according to the light intensity of the received light of the infrared light projected at the second light projection timing to measure an oxygen saturation of the artery.

2. The oxygen saturation measuring device according to claim 1, wherein the processor is configured to:

based on the peak timing and the bottom timing of the acquired third pulse wave signal, calculate a heartbeat interval of a predicted pulsation of the third pulse wave signal expected to arrive and a predicted peak timing and a predicted bottom timing in the predicted pulsation, and
determine the first light projection timing and the second light projection timing in the predicted pulsation, based on the predicted peak timing and the predicted bottom timing that are calculated.

3. The oxygen saturation measuring device according to claim 2, wherein the processor is configured to:

determine the first light projection timing and the second light projection timing in each of
a first maximum side light projection period from a timing at which 2/3 of the heartbeat interval of the predicted pulsation elapses from the peak timing in a pulsation immediately before the predicted pulsation, to the predicted peak timing, and
a first minimum side light projection period from the predicted bottom timing of the predicted pulsation to a timing at which 1/3 of the heartbeat interval of the predicted pulsation elapses from the predicted bottom timing.

4. The oxygen saturation measuring device according to claim 1, wherein the processor is configured to:

set a maximum side threshold value of an amplitude, based on a maximum amplitude of the third pulse wave signal acquired at the peak timing,
set a minimum side threshold value of the amplitude, based on a minimum amplitude of the third pulse wave signal acquired at the bottom timing, and
determine the first light projection timing and the second light projection timing in each of a second maximum side light projection period in which the amplitude is equal to or higher than the maximum side threshold value and a second minimum side light projection period in which the amplitude is equal to or lower than the minimum side threshold value, in the pulsation of the third pulse wave signal.

5. The oxygen saturation measuring device according to claim 4, wherein:

the maximum side threshold value is 2/3 or more of a difference between the maximum amplitude and the minimum amplitude, and
the minimum side threshold value is 1/3 or less of the difference between the maximum amplitude and the minimum amplitude.

6. The oxygen saturation measuring device according to claim 1, wherein the processor is configured to:

calculate a coefficient of variation representing a state of pulsation based on a heartbeat interval of the acquired third pulse wave signal,
execute processing 1 in a case in which the calculated coefficient of variation is equal to or less than a preset threshold value, and
execute processing 2 in a case in which the coefficient of variation exceeds the threshold value,
processing 1 being processing of:

based on the peak timing and the bottom timing of the acquired third pulse wave signal, calculating a heartbeat interval of a predicted pulsation of the third pulse wave signal expected to arrive and a predicted peak timing and a predicted bottom timing in the predicted pulsation, and
determining the first light projection timing and the second light projection timing in the predicted pulsation, based on the predicted peak timing and the predicted bottom timing that are calculated, and
processing 2 being processing of:

setting a maximum side threshold value of an amplitude, based on a maximum amplitude of the third pulse wave signal acquired at the peak timing,
setting a minimum side threshold value of the amplitude, based on a minimum amplitude of the third pulse wave signal acquired at the bottom timing, and
determining the first light projection timing and the second light projection timing in each of a second maximum side light projection period in which the amplitude is equal to or higher than the maximum side threshold value and a second minimum side light projection period in which the amplitude is equal to or lower than the minimum side threshold value, in the pulsation of the third pulse wave signal.

7. The oxygen saturation measuring device according to claim 6, wherein a threshold value of the coefficient of variation is 0.1 or less.

8. The oxygen saturation measuring device according to any one of claims 1 to 7, which is a wearable device that is wearable by a subject.

9. An oxygen saturation measuring method comprising:

projecting, to an artery, reference light having a wavelength at which an absorption coefficient of oxidized hemoglobin and an absorption coefficient of reduced hemoglobin are higher than those of red light and infrared light;
receiving, as received light, transmitted light or reflected light corresponding to the projected reference light;
acquiring a third pulse wave signal corresponding to a light intensity of the received light of the reference light;
based on a peak timing and a bottom timing of the acquired third pulse wave signal in one pulsation, determining a first light projection timing at which the red light is projected and a second light projection timing at which the infrared light is projected;
projecting the red light at the determined first light projection timing;
projecting the infrared light at the determined second light projection timing; and
calculating oxygen saturation based on a first pulse wave signal acquired according to a light intensity of the received light of the transmitted light or the reflected light corresponding to the red light projected at the first light projection timing and a second pulse wave signal acquired according to a light intensity of the received light of the transmitted light or the reflected light corresponding to the infrared light projected at the second light projection timing to measure an oxygen saturation of the artery.

10. An oxygen saturation measuring program for causing a processor to execute processing of:

projecting, to an artery, reference light having a wavelength at which an absorption coefficient of oxidized hemoglobin and an absorption coefficient of reduced hemoglobin are higher than those of red light and infrared light;
receiving, as received light, transmitted light or reflected light corresponding to the projected reference light;
acquiring a third pulse wave signal corresponding to a light intensity of the received light of the reference light;
based on a peak timing and a bottom timing of the acquired third pulse wave signal in one pulsation, determining a first light projection timing at which the red light is projected and a second light projection timing at which the infrared light is projected;
projecting the red light at the determined first light projection timing;
projecting the infrared light at the determined second light projection timing; and

calculating oxygen saturation based on a first pulse wave signal acquired according to a light intensity of the received light of the transmitted light or the reflected light corresponding to the red light projected at the first light projection timing and a second pulse wave signal acquired according to a light intensity of the received light of the transmitted light or the reflected light corresponding to the infrared light projected at the second light projection timing to measure an oxygen saturation of the artery.

FIG.1

# FIG.2

# FIG.3

| | | |
|---|---|---|
| 21 — CPU | | STORAGE — 24 |
| 22 — ROM | | USER INTERFACE — 25 |
| 23 — RAM | | COMMUNICATION INTERFACE — 26 |

20

27

# FIG.4

START

PROJECT REFERENCE LIGHT TO ARTERY — S1

RECEIVE RECEIVED LIGHT CORRESPONDING TO REFERENCE LIGHT — S2

ACQUIRE THIRD PULSE WAVE SIGNAL CORRESPONDING TO RECEIVED LIGHT OF REFERENCE LIGHT — S3

DETERMINE FIRST LIGHT PROJECTION TIMING AND SECOND LIGHT PROJECTION TIMING BASED ON PEAK TIMING AND BOTTOM TIMING OF THIRD PULSE WAVE SIGNAL — S4

PROJECT RED LIGHT AT FIRST LIGHT PROJECTION TIMING AND PROJECT INFRARED LIGHT AT SECOND LIGHT PROJECTION TIMING — S5

ACQUIRE EACH OF FIRST PULSE WAVE SIGNAL CORRESPONDING TO RED LIGHT PROJECTED AT FIRST LIGHT PROJECTION TIMING AND SECOND PULSE WAVE SIGNAL CORRESPONDING TO INFRARED LIGHT PROJECTED AT SECOND LIGHT PROJECTION TIMING — S6

CALCULATE OXYGEN SATURATION BASED ON FIRST PULSE WAVE SIGNAL AND SECOND PULSE WAVE SIGNAL — S7

END

# FIG.5

# FIG.6

THIRD PULSE WAVE SIGNAL PS3 OF REFERENCE LIGHT

PREDICTED PULSATION

PEAK TIMING

HEARTBEAT INTERVAL

TM   TM   TM   TM   TEM

BOTTOM TIMING

HEARTBEAT INTERVAL

Tm   Tm   Tm   Tm   TEm

LIGHT PROJECTION TIMINGS OF RED LIGHT AND INFRARED LIGHT

CURRENT TIME POINT

TF1   TF2   TF1   TF2

EP 4 606 304 A1

# FIG.7

THIRD PULSE WAVE
SIGNAL PS3 OF
REFERENCE LIGHT

PEAK TIMING

HEARTBEAT INTERVAL

TM     TM     TM     TM     TEM

BOTTOM TIMING

HEARTBEAT INTERVAL

Tm     Tm     Tm     Tm     TEm

LIGHT PROJECTION
TIMINGS OF RED
LIGHT AND
INFRARED LIGHT

CURRENT
TIME POINT   TF1   TF1
             TF2      TF2

EP 4 606 304 A1

# FIG.8

# FIG.9

THIRD PULSE WAVE SIGNAL PS3 OF REFERENCE LIGHT

MAXIMUM AMPLITUDE

MINIMUM AMPLITUDE

DM2

PREDICTED MAXIMUM AMPLITUDE

MAXIMUM SIDE THRESHOLD VALUE

MINIMUM SIDE THRESHOLD VALUE

PREDICTED MINIMUM AMPLITUDE

Dm2

TIMING AT WHICH AMPLITUDE EXCEEDS MAXIMUM SIDE THRESHOLD VALUE AND TIMING AT WHICH AMPLITUDE FALLS BELOW MINIMUM SIDE THRESHOLD VALUE

LIGHT PROJECTION TIMINGS OF RED LIGHT AND INFRARED LIGHT

CURRENT TIME POINT

TF1    TF1

TF2    TF2

EP 4 606 304 A1

FIG.10A

PREDICTED
MAXIMUM
AMPLITUDE

MAXIMUM SIDE
THRESHOLD VALUE

CLOSEST MINIMUM
AMPLITUDE POINT

DM2

FIG.10B

CLOSEST MAXIMUM
AMPLITUDE POINT

MINIMUM SIDE
THRESHOLD
VALUE

PREDICTED
MINIMUM
AMPLITUDE

Dm2

# FIG.11

THIRD PULSE WAVE
SIGNAL OF
REFERENCE LIGHT

MAXIMUM
AMPLITUDE

DM2

PREDICTED
MAXIMUM
AMPLITUDE

MAXIMUM SIDE
THRESHOLD VALUE

MINIMUM SIDE
THRESHOLD VALUE

PREDICTED
MINIMUM
AMPLITUDE

MINIMUM AMPLITUDE

TIMING AT WHICH AMPLITUDE EXCEEDS
MAXIMUM SIDE THRESHOLD VALUE
AND TIMING AT WHICH AMPLITUDE FALLS
BELOW MINIMUM SIDE THRESHOLD VALUE

Dm2

LIGHT PROJECTION
TIMINGS OF RED LIGHT
AND INFRARED LIGHT

CURRENT
TIME POINT

TF1    TF1
TF2    TF2

FIG.12

FIG.13

## FIG.14A

RED LIGHT

## FIG.14B

INFRARED LIGHT

## FIG.14C

REFERENCE LIGHT

## INTERNATIONAL SEARCH REPORT

| International application No. |
| --- |
| **PCT/JP2023/036931** |

| A. | CLASSIFICATION OF SUBJECT MATTER |
| --- | --- |

*A61B 5/1455*(2006.01)i
FI:  A61B5/1455

According to International Patent Classification (IPC) or to both national classification and IPC

| B. | FIELDS SEARCHED |
| --- | --- |

Minimum documentation searched (classification system followed by classification symbols)

A61B5/145

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | JP 2022-117113 A (SEIKO EPSON CORP) 10 August 2022 (2022-08-10) paragraphs [0008]-[0104], fig. 1-10 | 1-5, 8-10 |
| A | | 6-7 |
| Y | JP 2020-130875 A (FUKUDA DENSHI KK) 31 August 2020 (2020-08-31) paragraphs [0012]-[0041], fig. 1-6 | 1-5, 8-10 |

☐ Further documents are listed in the continuation of Box C.  ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **27 October 2023** | **19 December 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/036931**

| Patent document cited in search report | | | Publication date (day/month/year) | Patent family member(s) | Publication date (day/month/year) |
|---|---|---|---|---|---|
| JP | 2022-117113 | A | 10 August 2022 | US 2022/0240822 A1 paragraphs [0017]-[0131], fig. 1-10 | |
| JP | 2020-130875 | A | 31 August 2020 | (Family: none) | |

Form PCT/ISA/210 (patent family annex) (January 2015)

**EP 4 606 304 A1**

## REFERENCES CITED IN THE DESCRIPTION

**Patent documents cited in the description**

- JP 2008167868 A

- JP 2022167840 A